# EUROPEAN PATENT APPLICATION

(11) **EP 4 813 383 A1**
(43) Date of publication of application: **30.09.2026**
(21) Application number: 25789928.6
(22) Date of filing: 16.04.2025
(51) Int. Cl.: A61K 31/423, A61P 7/06, A61P 9/10, A61P 17/02, A61P 13/12, A61P 9/00, A61P 31/00, A61P 29/00, A61P 35/00, A61K 31/44, A61K 31/445, A61K 31/495, A61K 31/4406, A61K 31/4409, A61K 31/439

(54) **USE OF TAFAMIDIS AND PHARMACEUTICALLY ACCEPTABLE SALT THEREOF IN PREVENTION, ALLEVIATION, OR TREATMENT OF DISEASES ASSOCIATED WITH HIF-2a**

(30) Priority: 19.04.2024 CN 202410474210
(71) Applicant: Huaya Jiyuan (Shenzhen) Pharmaceutical Co., Ltd., Shenzhen, Guangdong 518122 (CN)
(72) Inventor: XUE, Xiaoqian, Shenzhen, Guangdong 518122 (CN); ZHONG, Fengxia, Shenzhen, Guangdong 518122 (CN); WEN, Yuanmei, Shenzhen, Guangdong 518122 (CN); LI, Chao, Shenzhen, Guangdong 518122 (CN); LI, Shuo, Shenzhen, Guangdong 518122 (CN)
(74) Representative: Metida
(86) International application number: PCT/CN2025/089203
(87) International publication number: WO 2025/218683

(57) **Abstract**

This application relates to the technical field of biomedicine, and in particular, to a use of tafamidis or a salt thereof in preventing, alleviating, or treating HIF-2α-related diseases. Through in-vitro experiments, this application confirms that tafamidis enhances transcriptional activity of HIF-2α, thereby promoting the expression of downstream genes regulated by HIF-2α; and further confirms, in an aristolochic acid-induced zebrafish chronic kidney disease model, a cisplatin-induced acute kidney injury and renal anemia model, and a unilateral ureteral obstruction model, that tafamidis exerts effects of increasing erythropoiesis and alleviating renal injury. In addition, this application relates to a new use of a known drug ("drug repurposing"), thereby reducing drug development cost and treatment cost, and producing significant economic benefits and clinical application value.

## Description

### TECHNICAL FIELD

This application relates to the technical field of biomedicine, and in particular, to a use of tafamidis and a pharmaceutically acceptable salt thereof in preventing, alleviating, or treating HIF-2α-related diseases.

### BACKGROUND

Under hypoxic conditions, a body is capable of spontaneously eliciting hypoxic responses to maintain the oxygen acquisition capacity of the body. In 1992, Gregg L. Semenza et al. discovered a protein capable of specifically binding to a hypoxia response element (HRE) of an erythropoietin gene and affecting the expression of some genes. This protein is termed hypoxia-inducible factor (HIF) (Semenza GL et al., Mol. Cell Biol., 1992, 12, 5447-5454). The target genes of hypoxia-inducible factor (HIF) are highly diverse and can influence hematopoiesis, angiogenesis, iron ion transport, glucose utilization, resistance-to-oxidative stress, cell differentiation, cell survival and apoptosis, extracellular matrix homeostasis, and tumorigenesis of the body. HIF is a heterodimer formed of α and β subunits. The α subunit is a functional subunit, and is highly sensitive to changes in intracellular oxygen concentration, and is tightly regulated, thereby adjusting HIF activity. The β subunit is a structural subunit, also known as aryl hydrocarbon receptor nuclear translocator (ARNT), and is stably expressed in cells. The mRNA transcription and protein expression levels of the β subunit are not affected by oxygen concentration. Both the α and β subunits of HIF belong to the basic helix-loop-helix (bHLH) transcription factor superfamily. Human HIF-α includes three isoforms: HIF-1α, HIF-2α, and HIF-3α. HIF-1α is ubiquitously distributed in vivo and plays an important role in angiogenesis induced by local tissue ischemia or hypoxia, but exerts little effect on iron metabolism. HIF-2α exhibits a restricted distribution and plays an important role in the expression and synthesis of erythropoietin (EPO) genes in renal tissue, enhances intestinal iron absorption by upregulating the expression of duodenal cytochrome and divalent metal transporter-1, reduces hepatic antimicrobial peptide expression, and plays a dominant role in iron metabolism. Structurally different from other subtypes, HIF-3α lacks a DNA-binding domain and therefore cannot affect gene expression. Studies show that HIF-3α may play a negative regulatory role in HIF-mediated gene expression. Therefore, HIF-1α and HIF-2α play a specified role in a hypoxic response process. In a mouse test involving knockout of HIF-1α and HIF-2α genes, the necessity of HIF-1α and HIF-2α in the hypoxic response process is confirmed. However, in the development of compounds for treating chronic renal anemia, changes in HIF-2α are more important than those in HIF-1α.

Both the HIF-2α subunit and the ARNT subunit belong to the Per-ARNT-Sim (PAS) subfamily of the bHLH family, and are structurally similar, and mainly include an N-terminal bHLH domain (DNA bonding domain, DBD) and two adjacent PASA and PASB domains (ligand bonding domains, LBDs). The C-terminus binds to transcriptional coactivators to regulate transcription of downstream genes. Studies have found that there is a cavity of approximately 290 Å³ within the PASB domain of the HIF-2α subunit. The cavity in combination with a modulator can influence heterodimerization between the HIF-2α subunit and the ARNT subunit, thereby blocking or activating DNA binding and transcription of target genes. Downstream target genes of HIF-2α include vascular endothelial growth factor (VEGF), erythropoietin (EPO), cyclin 1 (Cyclin1), glucose transporter 1 (GLUT1), and the like. Such genes are associated with kidney diseases, renal anemia, cardiovascular diseases, infections, cancers, and the like.

Damage to renal tissue in patients with kidney diseases may lead to insufficient EPO and impaired iron homeostasis, thereby causing renal anemia. Renal anemia not only severely reduces the quality of life of patients, but is also an important factor contributing to the occurrence of cardiovascular diseases and increased mortality. Recombinant human erythropoietin (rHuEPO) or erythropoiesis-stimulating agents (ESAs) can be used to treat renal anemia by increasing hemoglobin levels. However, in clinical trials, a relatively high hemoglobin target level is positively correlated with the risk of cardiovascular adverse events. Currently, an emerging therapy for renal anemia is to pharmacologically inhibit prolyl hydroxylase domain enzymes (PHDs) to stabilize HIF-2 protein, thereby stimulating endogenous EPO production in renal or non-renal tissues. However, excessive upregulation of HIF-1α by the PHD inhibitor promotes inflammatory pathways, accelerates cardiac and renal injury, and consequently increases the risk of pulmonary hypertension and inflammation. The activation of HIF-2α exerts protective effects. Therefore, selective agonists of HIF-2α may offer greater therapeutic advantages.

A typical pathological change in kidney diseases is renal fibrosis. Kyoung HK et al. found that long-term activation of HIF-2α helps suppress renal fibrosis and improve renal function. Yu et al. found that in the early stage of administration of the PHD inhibitor L-mimosine, selective activation of HIF-1α leads to increased expression of fibrotic factor CTGF and phosphorylated Smad, aggravates macrophage infiltration and fibrosis in renal tissue; however, in the middle and late stages, predominant activation of HIF-2α upregulates EPO and VEGF expression and alleviates renal injury. Qu et al. confirmed that knockout of HIF-2α results in more severe renal injury. In summary, in acute and chronic renal injury, excessive activation of HIF-1α may exacerbate renal injury whereas HIF-2α plays a protective role.

Tafamidis (2-(3,5-dichlorophenyl)-1,3-benzoxazole-6- carboxylic acid) and tafamidis meglumine (the meglumine salt of 1-deoxy-1-(methylamino)-D-glucitol salt of 2-(3,5-dichlorophenyl)-1,3-benzoxazole-6-carboxylic acid, also known as tafamidis glucamine salt) are transthyretin stabilizers, originally developed and produced by Pfizer. Vyndamax and Vyndaqel are both capsules for oral administration and contain tafamidis as an active moiety. The U.S. Food and Drug Administration (FDA) has approved Vyndaqel and Vyndamax for the treatment of wild-type or hereditary transthyretin-mediated amyloid cardiomyopathy in adults to reduce cardiovascular mortality and cardiovascular-related hospitalization. The European Medicines Agency (EMA) has approved Vyndaqel for the treatment of transthyretin amyloidosis in adult patients with stage I symptomatic polyneuropathy to delay peripheral neurological impairment.

Tafamidis has been used clinically for many years, and its safety has been established. Therefore, the development of additional therapeutic uses therefor can further benefit humankind.

### SUMMARY

This application aims to provide a use of tafamidis, an analog thereof, and a salt thereof in preventing, alleviating, or treating HIF-2α-related diseases to implement drug repurposing, reduce drug development cost and treatment cost, and produce significant economic benefits and clinical application value.

According to a first aspect, this application provides a use of tafamidis or a pharmaceutically acceptable salt thereof, or a pharmaceutical composition containing tafamidis or a pharmaceutically acceptable salt thereof in preparing a drug for preventing, alleviating, or treating diseases associated with HIF-2α activity.

In some embodiments, the pharmaceutically acceptable salt of tafamidis is an acid addition salt or a base addition salt.

In some embodiments, the pharmaceutically acceptable salt of tafamidis is at least one selected from the following structures: 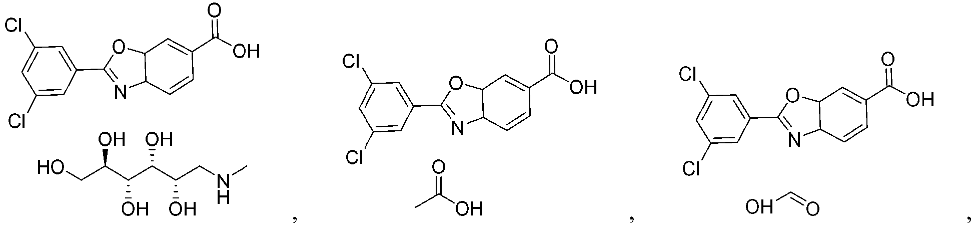 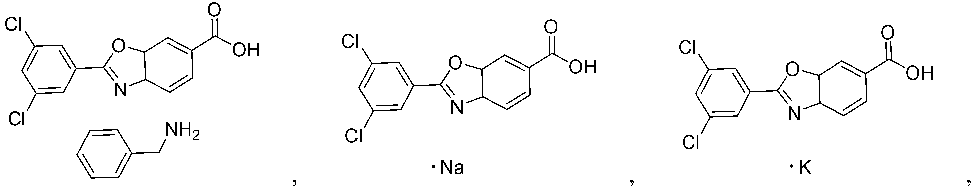 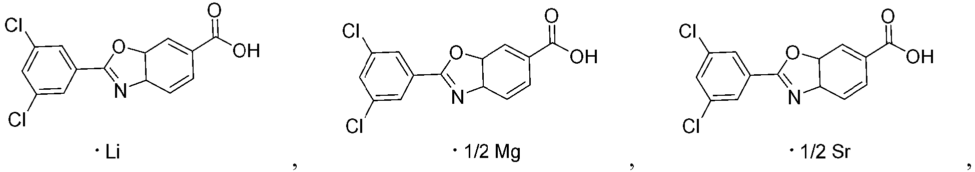 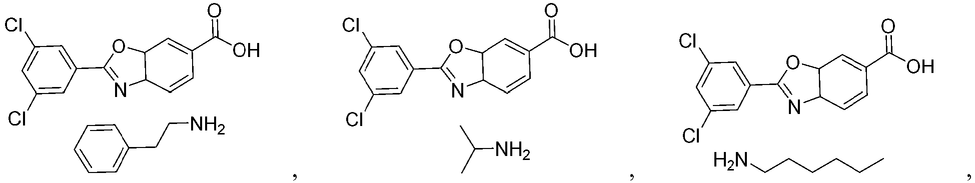 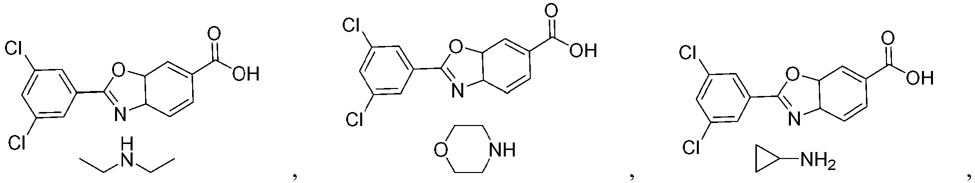 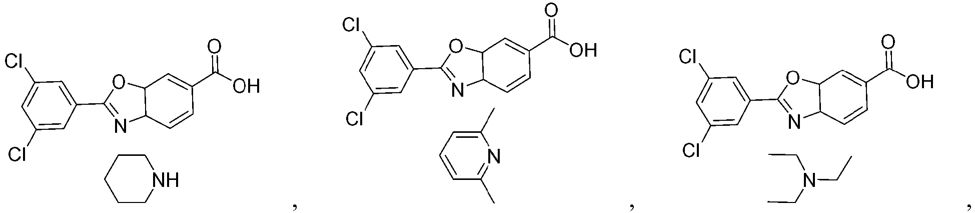 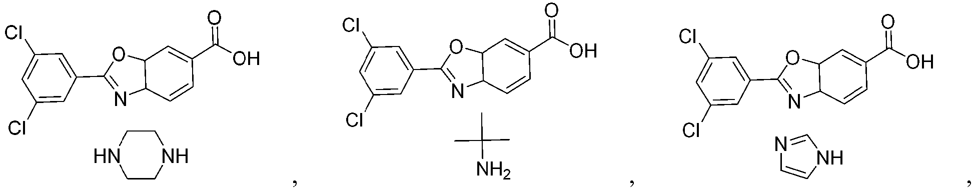 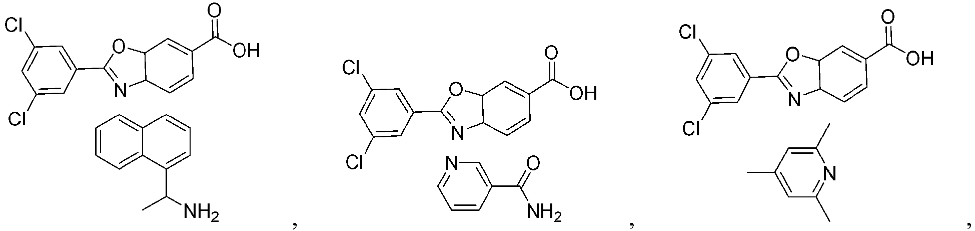 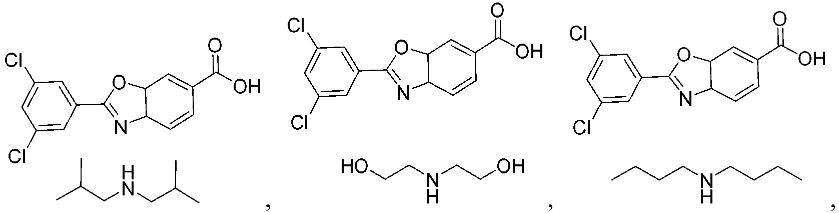 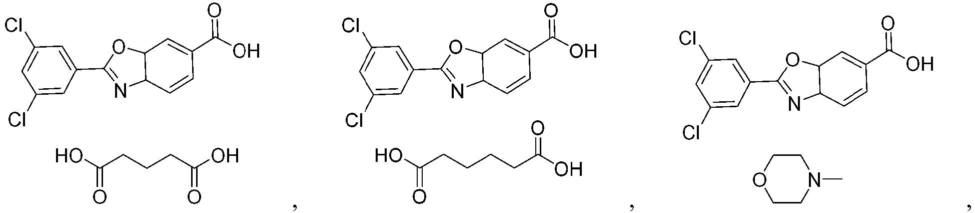 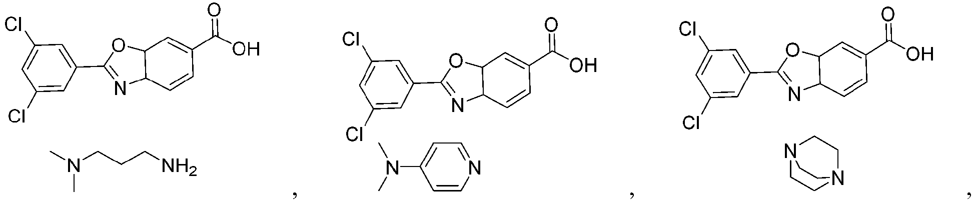 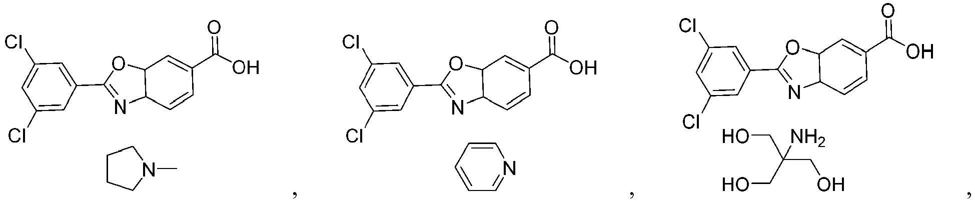 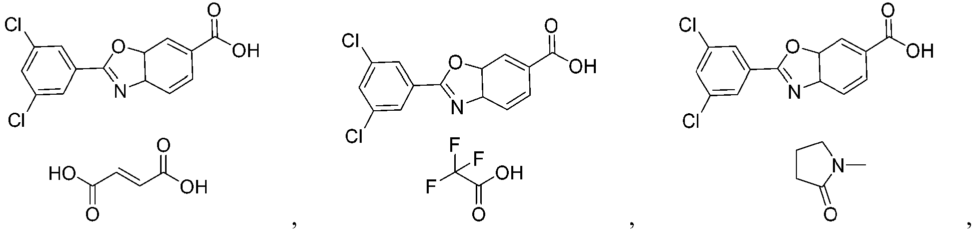 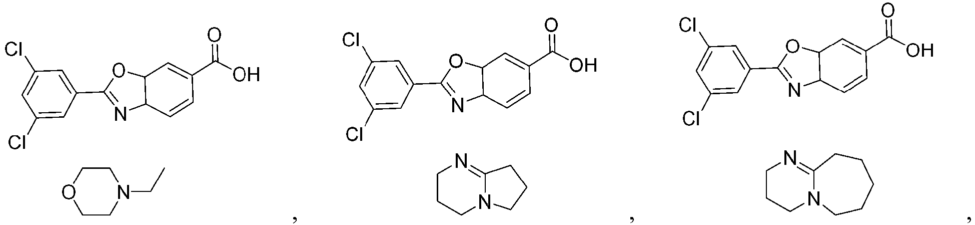 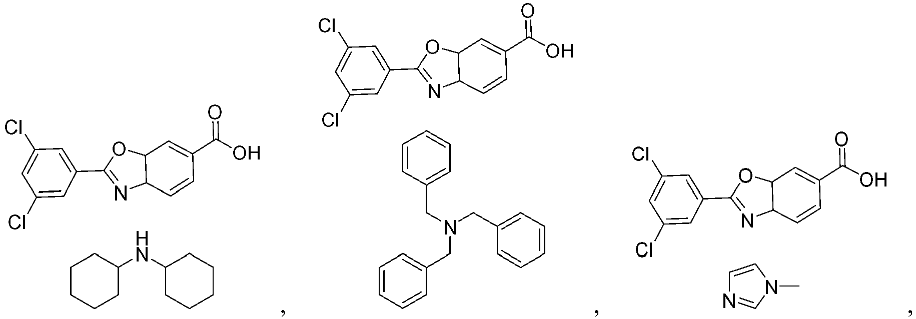 and

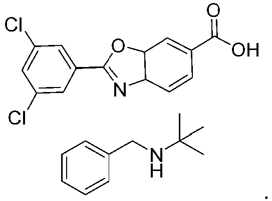

In some embodiments, the pharmaceutically acceptable salt of tafamidis is tafamidis meglumine.

In some embodiments, the pharmaceutical composition further includes a pharmaceutically acceptable excipient.

In some embodiments, the disease associated with HIF-2α activity includes hematopoietic disorders, anemia, postoperative surgery-related ischemia and sequelae thereof, postoperative wound healing, chronic kidney diseases, cardiovascular diseases, infections, inflammatory diseases, cancers, impairment of health status occurring during cancer treatment, or sequelae of acute and prolonged cerebral ischemia.

In some embodiments, the disease associated with HIF-2α activity includes: renal anemia; primary anemia; anemia associated with a neoplastic disease; chemotherapy-induced anemia; blood-loss-induced anemia; iron-deficiency-induced anemia; vitamin-deficiency-induced anemia; hypoplastic and aplastic anemia; hemolytic anemia; anemia caused by impaired iron utilization (anemia caused by iron utilization failure) or due to other endocrine disorders (such as hypothyroidism); ischemia and sequelae thereof caused by cardiac interventions using a heart-lung machine (such as bypass surgery and heart valve transplantation), carotid interventions, aortic interventions, and interventions involving opening or penetrating a skull using an instrument; primary glomerulonephritis; hypertensive renal arteriolosclerosis; diabetic nephropathy; secondary glomerulonephritis; tubulointerstitial diseases (chronic pyelonephritis, chronic uric acid nephropathy, obstructive nephropathy, drug-induced nephropathy, and the like); ischemic nephropathy; hereditary nephropathy (polycystic kidney, hereditary nephritis); cardiac insufficiency; coronary heart disease; angina pectoris; myocardial infarction; stroke; arteriosclerosis; primary, pulmonary and malignant hypertension and peripheral arterial occlusive disease; HIV infection; rheumatoid arthritis; or diseases of a rheumatic spectrum or other disease forms considered as autoimmune diseases following the use of cytostatics, antibiotics, or radiotherapy; and impairment of health status occurring during pharmacological treatment of such diseases (such as stroke and birth asphyxia).

In some embodiments, the disease associated with HIF-2α activity includes anemia, ischemia, vascular diseases, angina pectoris, myocardial infarction, metabolic disorders, or cancers.

In some embodiments, the disease associated with HIF-2α activity includes renal anemia and/or nephropathy.

In some embodiments, the tafamidis or a pharmaceutically acceptable salt thereof, or a pharmaceutical composition containing tafamidis or a pharmaceutically acceptable salt thereof, achieves prevention, alleviation, or treatment of diseases associated with HIF-2α activity by upregulating downstream VEGF genes regulated by HIF-2α.

In some embodiments, the tafamidis or a pharmaceutically acceptable salt thereof, or a pharmaceutical composition containing tafamidis or a pharmaceutically acceptable salt thereof, achieves prevention, alleviation, or treatment of diseases associated with HIF-2α activity by upregulating downstream EPO genes regulated by HIF-2α.

According to another aspect, this application provides a combination therapy, where the tafamidis or a pharmaceutically acceptable salt thereof, or the pharmaceutical composition containing tafamidis or a pharmaceutically acceptable salt thereof is further combined with a prolyl hydroxylase inhibitor for use in preparing a drug for preventing, alleviating, or treating diseases associated with HIF-2α activity.

### Beneficial effects:

Compared with the prior art, an embodiment of this application achieves at least one of the following beneficial effects:
(1) Through RT-qPCR, it is determined in this application that the tafamidis can upregulate the downstream VEGF and EPO genes regulated by HIF-2α. These results indicate that tafamidis can effectively activate expression of downstream target genes of HIF-2α. Such activation may have potential therapeutic value for treating diseases such as ischemic diseases or anemia.
(2) In an aristolochic acid-induced zebrafish chronic kidney disease model, it is confirmed that tafamidis exerts effects of increasing erythropoiesis and alleviating renal injury, indicating that tafamidis exerts a protective effect on the kidney and a significant effect on alleviating renal anemia.
(3) In a 5/6 nephrectomy rat model, tafamidis increases the red blood cell count in chronic kidney disease rats, demonstrating potential value for the treatment of renal anemia. Comparison with roxadustat further indicates that tafamidis achieves a favorable balance between the erythropoiesis-promoting effect and the renal safety.
(4) In a cisplatin-induced kidney injury and renal anemia model, tafamidis significantly increases the red blood cell count, hemoglobin level, and hematocrit in rats with renal anemia, exhibiting potential for treating renal anemia. Urinalysis and omics-based analysis reveal that tafamidis can repair kidney injury, particularly by alleviating renal fibrosis.
(5) In a unilateral-ureteral-obstruction-induced kidney injury model, tafamidis exhibits strong efficacy in treating kidney injury and alleviating ischemia.
(6) Luciferase reporter gene assays demonstrate that both tafamidis and tafamidis meglumine significantly enhance the transcriptional activity of HIF-2α. Therefore, tafamidis and a salt thereof can be used to prevent and/or treat diseases associated with HIF-2α activity.
(7) Through in-vitro experiments, this application confirms that tafamidis enhances transcriptional activity of HIF-2α, thereby promoting the expression of downstream genes regulated by HIF-2α; and further confirms, in an aristolochic acid-induced zebrafish chronic kidney disease model, a cisplatin-induced acute kidney injury and renal anemia model, and a unilateral ureteral obstruction model, that tafamidis exerts effects in increasing erythropoiesis and alleviating renal injury. Through a series of experiments, this application verifies that tafamidis and a salt thereof can be used to prevent and/or treat diseases associated with HIF-2α activity, particularly renal anemia or nephropathy.

### Terminology

Some embodiments of this application are described below in detail, with examples illustrated by the accompanying chemical formulas. This application is intended to encompass all substitutions, modifications, and equivalent technical solutions, which are all included within the scope of the protection of this application defined in the claims. Those skilled in the art are aware that a variety of methods and materials similar or equivalent to those mentioned herein are available for implementing this application. This application is in no way limited to the methods and materials described herein. In the event that one or more of the documents, patents, and other similar materials used as a reference herein differ from or contradict this application (including but not limited to the defined terms, term applications, the described technologies, and the like), this application shall prevail.

It is further appreciated that some features of this application, which, for clarity, are described in a plurality of independent embodiments, may also be provided in combination in a single embodiment. Conversely, various features of this application, which, for brevity, are described in a single embodiment, may also be provided separately or in any suitable subcombination.

Unless otherwise specified, all scientific and technical terms used herein bear the same meanings as what are commonly understood by a person skilled in the technical field of this application. All patents and publications related to this application are incorporated herein by reference in their entirety.

In the context of this specification, reference to "one embodiment", "some embodiments", "one or more embodiments", "an embodiment", "specific example", "some examples", and the like means that a particular feature, structure, material, or characteristic described in connection with the embodiment or example is included in at least one embodiment or example of this application. In this embodiment, the appearances of such terms are not necessarily referring to the same embodiment or example. Moreover, the specific features, structures, materials, or characteristics described may be combined in any suitable manner in any one or more embodiments or examples. Furthermore, to the extent that no mutual conflict occurs, those skilled in the art may combine and integrate different embodiments or examples described in this specification, as well as features of different embodiments or examples.

In the following disclosure, all numerical values disclosed herein are approximate, regardless of whether words such as "about" or "approximately" are used. Each numerical value may deviate by 1%, 2%, 5%, 7%, 8%, 10%, 15%, 20%, or the like. Whenever a numerical value N is disclosed, any value of N±1%, N±2%, N±3%, N±5%, N±7%, N±8%, N±10%, N±15%, or N±20% is expressly disclosed, where "±" means plus or minus.

### BRIEF DESCRIPTION OF DRAWINGS

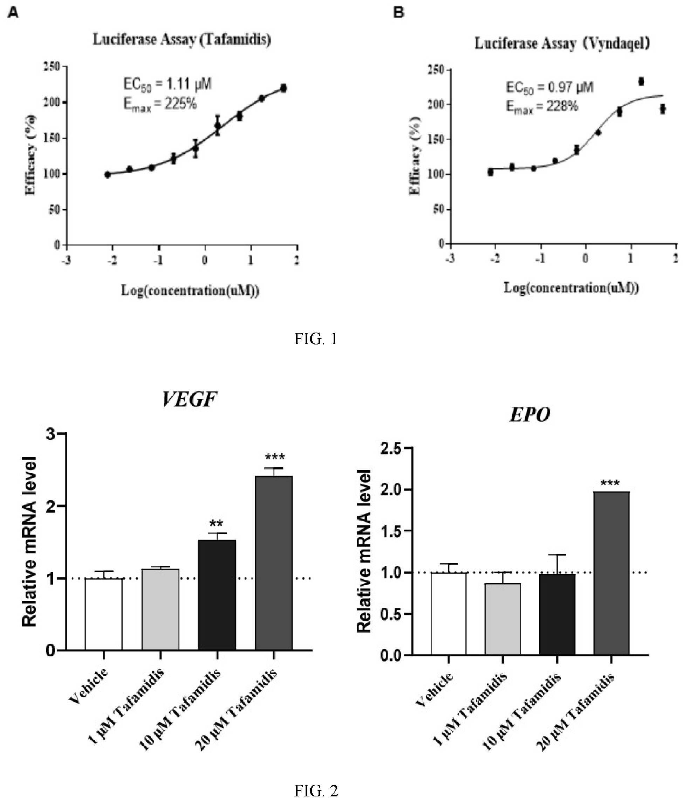

FIG. 1 is a graph illustrating luciferase reporter gene assay results according to Embodiment 1 of this application. In the figure, panel A shows an agonistic activity of tafamidis on HIF-2 protein, and panel B shows agonistic activity of tafamidis meglumine (B) on HIF-2 protein.
FIG. 2 is a graph illustrating real-time quantitative PCR-based assay results according to Embodiment 2 of this application. The results show that, in Embodiment 2 of this application, real-time reverse transcription quantitative polymerase chain reaction (RT-qPCR) detects that tafamidis upregulates transcription of downstream VEGF and EPO target genes of HIF-2.

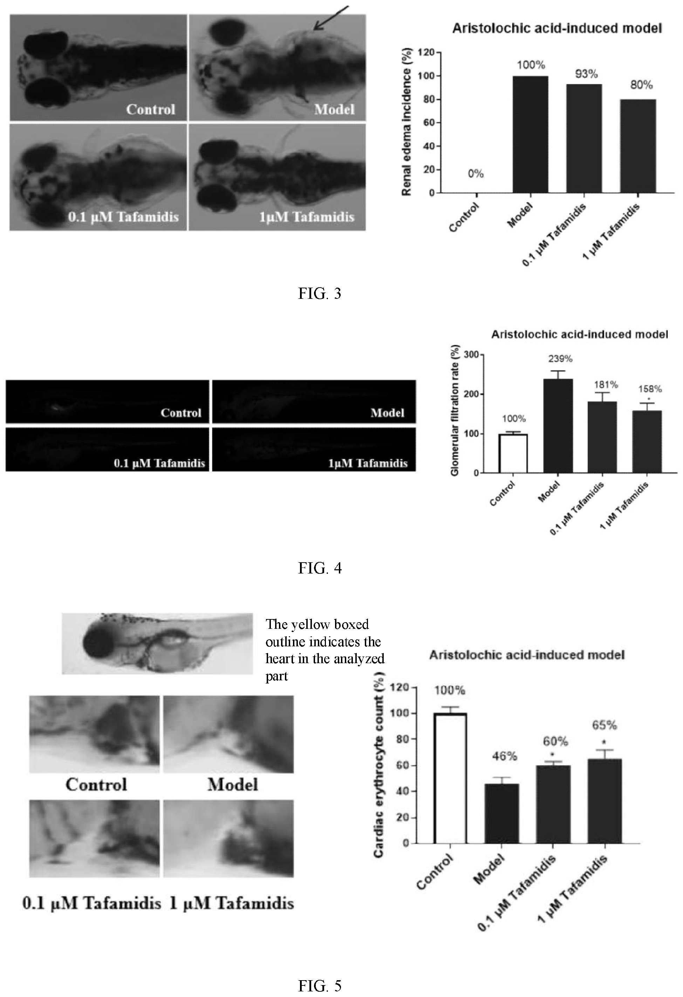

FIG. 3 shows test results of improving an incidence of renal edema according to Embodiment 3. In Embodiment 3 of this application, tafamidis improves renal edema in an aristolochic acid-induced kidney injury zebrafish model. In the figure, "control" represents a normal control group, "model" represents a model control group, "aristolochic acid-induced model" represents a model induced by aristolochic acid.
FIG. 4 shows test results of reducing glomerular filtration according to Embodiment 3. In Embodiment 3 of this application, tafamidis improves glomerular filtration in an aristolochic acid-induced kidney injury zebrafish model. In the figure, "control" represents a normal control group, "model" represents a model control group, and "aristolochic acid-induced model" represents a model induced by aristolochic acid.
FIG. 5 shows test results of improving renal anemia according to Embodiment 3. In Embodiment 3 of this application, tafamidis increases cardiac erythrocyte signal intensity in an aristolochic acid-induced kidney injury zebrafish model. In the figure, "control" represents a normal control group, "model" represents a model control group, and "aristolochic acid-induced model" represents a model induced by aristolochic acid.

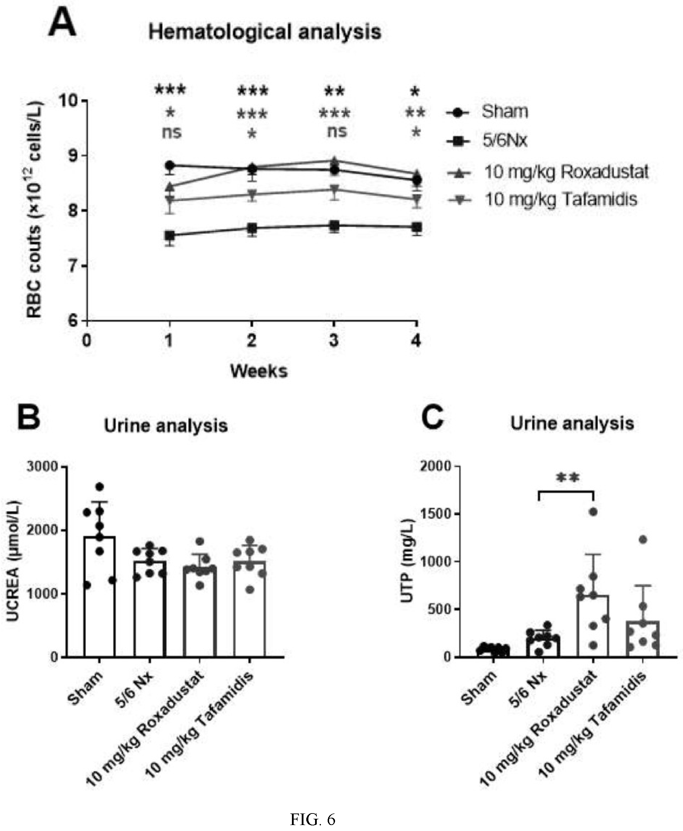

FIG. 6 shows test results according to Embodiment 4. In Embodiment 4 of this application, in a 5/6 nephrectomy rat model, tafamidis increases the red blood cell count (A) in chronic kidney disease rats. Urine analysis shows that tafamidis stabilizes creatinine levels (B) and urinary protein levels (C).

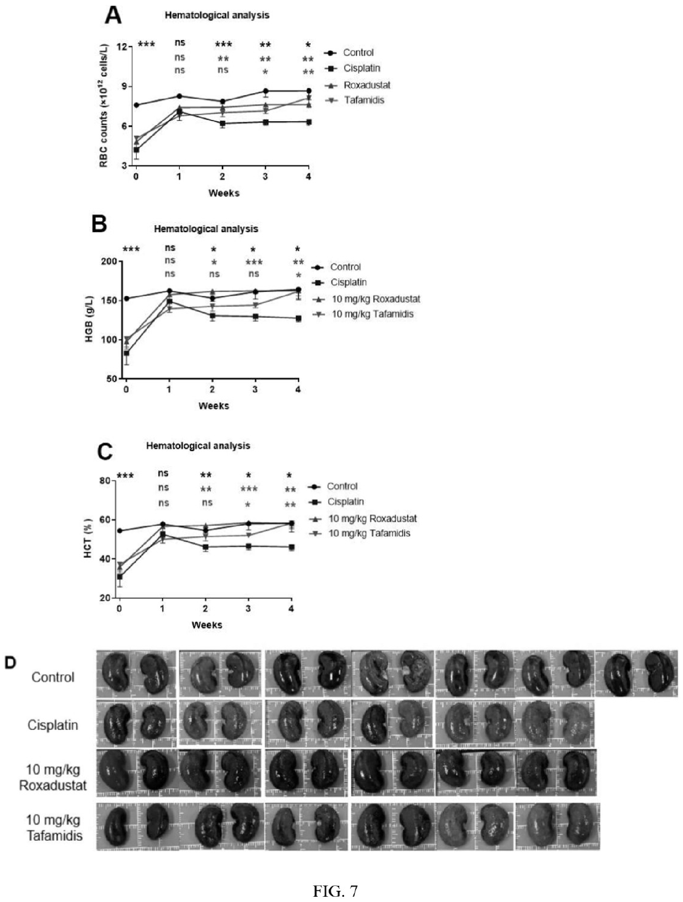

FIG. 7 shows results of hematological analysis according to Embodiment 5. In Embodiment 5 of this application, in a cisplatin-induced kidney injury and renal anemia model, tafamidis significantly increases the red blood cell count (A), hemoglobin level (B), and hematocrit (C) in rats with renal anemia. Renal tissue images of cisplatin-induced rats show that tafamidis significantly increases renal blood flow.

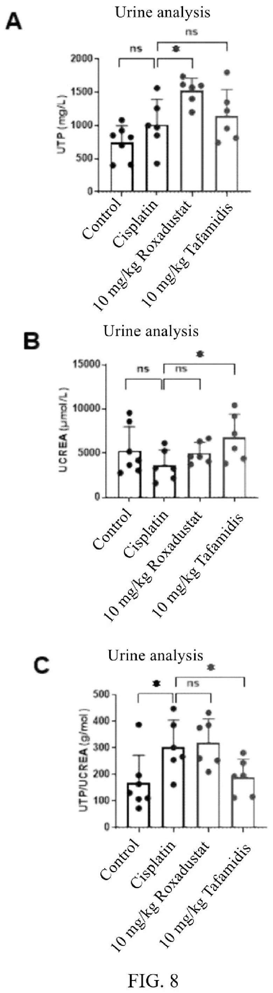

FIG. 8 shows results of urine analysis according to Embodiment 5. In Embodiment 5 of this application, in a cisplatin-induced kidney injury and renal anemia model, the urine analysis results are obtained. Tafamidis exerts little effect on UTP (A), significantly increases the UCREA level (B), and reduces the UTP/UCREA ratio (p < 0.05) (FIG. 8C), indicating that tafamidis exerts a significant effect in alleviating kidney injury.

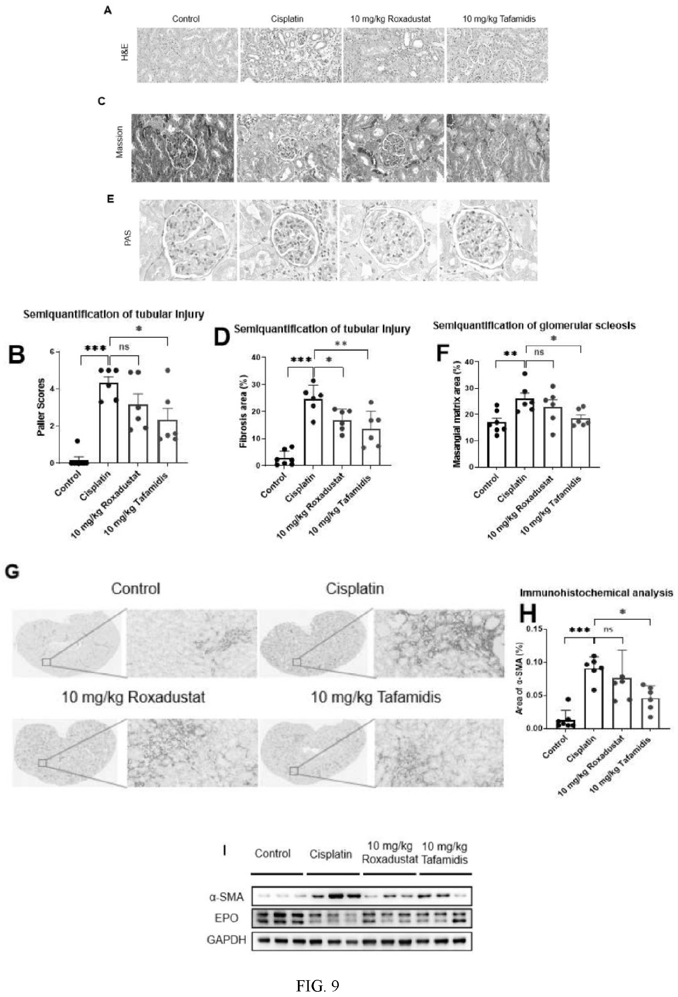

FIG. 9 shows renal pathological changes according to Embodiment 5. In Embodiment 5 of this application, in a cisplatin-induced kidney injury and renal anemia model, tafamidis demonstrates strong therapeutic effects in treating kidney injury and alleviating renal anemia. Panels A, C, E show images of kidney tissue: A: HE staining (40× magnification); C: Masson's trichrome staining (20× magnification); E: PAS staining (63× magnification); panels B and D show semiquantitative analysis of renal tubular injury in HE-stained (B) and Masson's trichrome-stained (D) sections; panel F shows semiquantitative analysis of glomerular sclerosis in a PAS-stained section; panel G shows a representative image of immunohistochemical staining of α-SMA protein expression (0.7× to 30× magnification); panel H shows semiquantitative analysis of α-SMA-positive regions; panel I shows western blot analysis of α-SMA and EPO protein expression amounts.

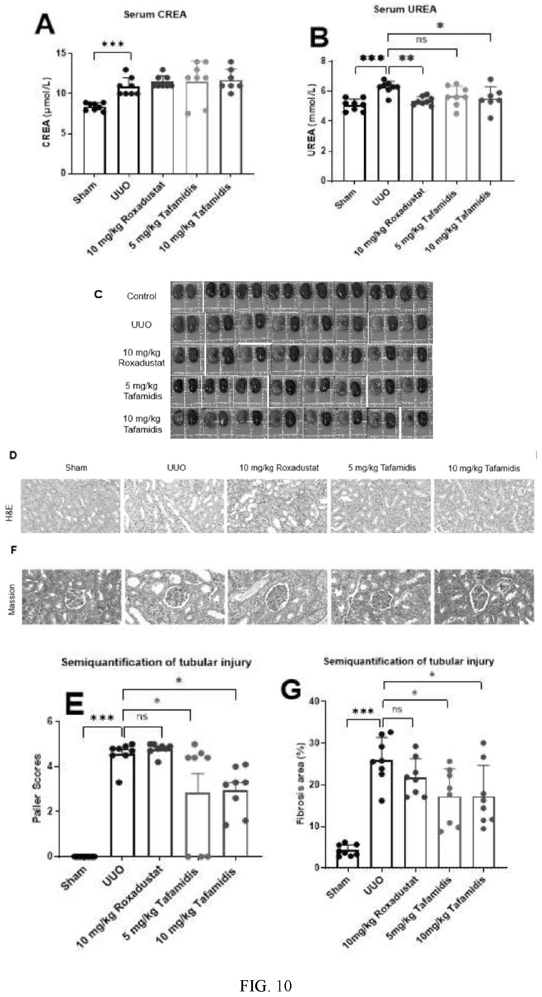

FIG. 10 shows test results according to Embodiment 6. In Embodiment 6 of this application, in a unilateral-ureteral-obstruction-induced kidney injury model, tafamidis exhibits strong efficacy in treating kidney injury and alleviating ischemia; serum biochemical analysis shows the effects of tafamidis on CREA (A) and UREA (B). Panel C shows a kidney tissue image (C) of a unilateral ureteral obstruction (UUO) model, indicating that tafamidis significantly alleviates renal ischemia; panels D and F show images of kidney tissue: D: HE staining (40× magnification); F: Masson's trichrome staining (20× magnification); panels E and G show semiquantitative analysis of renal tubular injury in HE-stained (E) and Masson's trichrome-stained (G) sections.

### DETAILED DESCRIPTION

To make the objectives, technical solutions, and advantages of this application clearer, this application is further described below in detail with reference to embodiments. The specific embodiments described herein are merely intended to explain this application without constituting any limitation on this application. In addition, in the following description, descriptions of well-known structures and technologies are omitted to avoid unnecessary confusion about the concepts of this application. Such structures and technologies are also described in many publications.

All reagents used in this application may be purchased from the market or prepared by using the method described in this application.

### Embodiment 1: Luciferase reporter gene assay

Experimental principles: 786-O is a renal carcinoma cell line that predominantly expresses HIF-2α and lacks functional HIF-1α. This characteristic makes 786-O cells an ideal model for studying HIF-2α transcriptional activity and related signaling pathways. In the luciferase reporter gene assay, a 786-O monoclonal cell line that stably expresses hypoxia response element (HRE)-driven luciferase (Luc) activity is obtained through puromycin selection. The HIF-2α agonist promotes dimerization of HIF-2α and HIF-β, and activates an HRE promoter. By measuring fluorescence intensity, luciferase activity can be determined, thereby assessing the ability of the HIF-2α agonist to promote transcription.

Experimental method: A total of 6000 stably transfected 786-O cells stably containing HRE and Luc sequences are seeded in each well of a 96-well plate, and 100 µL of RPMI-1640 culture medium containing 10% fetal bovine serum is added to each well. After 24 hours, a compound at a corresponding concentration is added to each well. A blank group is set as a control. Only a DMSO solvent is added in the well of the blank group. For initial compound screening, both tafamidis and tafamidis meglumine are tested at two concentrations: 2 µM and 20 µM. For concentration-gradient determination, each concentration is tested in triplicate. After co-incubation for 24 hours, the culture medium is discarded, and 20 µL of firefly luciferase reporter gene cell lysis buffer (RG126M, Beyotime) is added to each well. The 96-well plate is shaken on a microplate shaker for 10 minutes. After shaking, 10 µL of lysate is transferred into a white opaque plate, and then 10 µL of Steady-Lumi^{™} firefly luciferase assay reagent (RG058S, Beyotime) is added to each well. Finally, luminescence is measured using a microplate reader (Agilent Synergy Neo2). E₂₀ represents the effect of the compound at a 20 µM concentration, and is calculated as: E₂₀ = fluorescence value of the 20 µM well ÷ fluorescence value of the blank control × 100%. E₂ represents the effect of the compound at a 2 µM concentration, and is calculated as: E₂ = fluorescence value of the 2 µM well ÷ fluorescence value of the blank control × 100%. EC₅₀ is the compound concentration at which the agonistic efficacy is increased by 50% relative to the blank control. The agonistic efficacy of a compound is determined by calculating the concentration (EC₅₀) required to achieve a 50% increase in the fluorescence activity relative to the blank control and a maximum activation percentage (Eₘₐₓ). The maximum activation percentage (Eₘₐₓ) of an agonist refers to a ratio, expressed as a percentage, of the fluorescence value corresponding to the maximal effect (observed maximal response) induced when the agonist concentration reaches receptor-binding saturation in a particular receptor system, to the fluorescence value of the receptor system in the absence of agonist. In this experiment, Vyndaqel is directly used in place of tafamidis meglumine.

The test results show that the agonistic efficacy at a 20 µM concentration tafamidis is 207% while the agonistic efficacy at a 2 µM concentration is 142%, indicating that the agonistic efficacy of tafamidis increases with the increase of the concentration, exhibiting a clear dose dependence. As measured, EC₅₀ of tafamidis is 1.11 µM, and Eₘₐₓ is 225% (FIG. 1A), indicating that tafamidis exhibits strong agonistic activity and favorable efficacy.

The agonistic efficacy of tafamidis meglumine at a 20 µM concentration is 223% while the agonistic efficacy at a 2 µM concentration is 122%, indicating that the agonistic efficacy of the tafamidis meglumine increases with the increase of concentration, exhibiting a clear dose dependence. As measured, EC₅₀ of the tafamidis meglumine is 0.97 µM, and Eₘₐₓ is 228% (FIG. 1B), indicating that the tafamidis meglumine also exhibits strong agonistic activity and favorable efficacy.

As can be seen, tafamidis and a pharmaceutically acceptable salt thereof, serving as HIF-2α agonists, promote dimerization of HIF-2α and HIF-β and enhance bonding between HIF-2α and HIF-β, thereby exerting a protective effect of HIF-2α.

### Embodiment 2: Real-time reverse transcription quantitative polymerase chain reaction (qRT-PCR) assay

Experimental method: 786-O renal carcinoma cells are seeded into a 12-well plate. After 24 hours, tafamidis is added at different concentrations (1 µM, 10 µM, and 20 µM, separately), and the cells are co-incubated with the compound for 24 hours. No treatment is applied to the vehicle group. RNA is extracted using a TRIZOL reagent. cDNA transcription is performed using an All-in-one^{™} First-Strand cDNA Synthesis Kit (for specific operating procedures, see the manufacturer's instructions). A SYBR reagent is used for fluorescence signal detection, and GAPDH is used as an internal control. The qRT-PCR primers are as follows:
GAPDH_fwd, GCACCGTCAAGGCTGAGAAC;
GAPDH_rev, TGGTGAAGACGCCAGTGGA;
VEGF_fwd, AGGGCAGAATCATCACGAAGT;
VEGF_rev, AGGGTCTCGATTGGATGGCA;
EPO_fwd, AACAATCACTGCTGACACTT;
EPO_rev, AGAGTTGCTCTCTGGACAGT.

Test results (shown in FIG. 2): Relative to the vehicle, 10 µM tafamidis upregulates vascular endothelial growth factor (VEGF) gene expression by 0.5-fold, and 20 µM tafamidis upregulates VEGF gene expression by 1.4-fold (FIG. 2). Relative to the vehicle, 20 µM tafamidis upregulates erythropoietin (EPO) gene expression by 0.9-fold (FIG. 2). The tafamidis at different concentrations exhibits upregulatory effects on VEGF and EPO gene expression, indicating that tafamidis can effectively activate expression of downstream target genes of HIF-2α. Such activation may have potential therapeutic value for treating diseases such as ischemic diseases or anemia. Statistical analysis is performed using GraphPad Prism software (Version 8.0) and using two-tailed testing, *P < 0.05, **P < 0.01, ***P < 0.001.

### Embodiment 3: Aristolochic acid-Induced chronic kidney disease experiment in zebrafish

### 3.1 Improvement of the incidence of renal edema

Wild-type AB strain zebrafish at 2 days post-fertilization (2 dpf) are randomly selected and placed in a 6-well plate, with 30 zebrafish treated in each well (treatment group). Other than the normal control group, all treatment groups are exposed to aristolochic acid in aqueous solution to establish a zebrafish renal anemia model. After treatment at 28 °C for 18 hours, a normal control group and a model control group are set up simultaneously. Tafamidis samples are administered in aqueous solution to the treatment groups separately (at concentrations of 20 µM, 10 µM, and 1 µM separately, each concentration being tested in triplicate). Water is administered to the normal control group and the model control group correspondingly, with a volume of 3 mL per well. After continued treatment at 28 °C for 30 hours, each treatment group is observed under a dissecting microscope, the number of zebrafish exhibiting renal edema is counted, and the renal edema incidence (%) is calculated for each treatment group.

### 3.2 Reduction of glomerular filtration

Wild-type AB strain zebrafish at 2 days post-fertilization are randomly selected and placed in a 6-well plate, with 30 zebrafish treated in each well (treatment group). Other than the normal control group, all treatment groups are exposed to aristolochic acid in aqueous solution to establish a zebrafish renal anemia model. After treatment at 28 °C for 18 hours, a normal control group and a model control group are set up simultaneously. Tafamidis samples are administered in aqueous solution to the treatment groups separately (at concentrations of 0.1 µM and 1 µM separately). Water is administered to the normal control group and the model control group correspondingly, with a volume of 3 mL per well. After continued treatment at 28 °C for 4 hours, all treatment groups are intravenously injected with a fluorescent tracer (dextran tetramethylrhodamine). After a further 1 day of treatment at 28 °C, 10 zebrafish are randomly selected from each treatment group and photographed under a fluorescence microscope. Data are analyzed and collected using NIS-Elements D 3.20 advanced image processing software, and whole-body fluorescence intensity in the zebrafish is analyzed. Statistical analysis of this indicator is used to evaluate the efficacy of the samples in repairing renal injury. Statistical results are expressed as mean±SE. Statistical analysis is performed using SPSS 26.0 software, and p < 0.05 indicates a statistically significant difference.

### 3.3 Evaluation of efficacy in alleviating renal anemia (cardiac erythrocyte staining intensity)

Wild-type AB strain zebrafish at 2 days post-fertilization are randomly selected and placed in a 6-well plate, with 30 zebrafish treated in each well (treatment group). Other than the normal control group, all treatment groups are exposed to aristolochic acid in aqueous solution to establish a zebrafish renal anemia model. After treatment at 28 °C for 18 hours, a normal control group and a model control group are set up simultaneously. Tafamidis samples are administered in aqueous solution to the treatment groups separately (at concentrations of 0.1 µM and 1 µM separately). Water is administered to the normal control group and the model control group correspondingly, with a volume of 3 mL per well. After continued treatment at 28 °C for 30 hours, staining is performed using o-dianisidine. After staining, 10 zebrafish are randomly selected from each treatment group and photographed under a dissecting microscope. Data is collected using NIS-Elements D 3.20 advanced image processing software, and the staining intensity of cardiac erythrocytes in the zebrafish is analyzed. The statistical significance of this indicator is used to evaluate the efficacy of the sample in alleviating renal anemia. Statistical analysis is performed using SPSS 26.0 software, and p < 0.05 indicates a statistically significant difference.

### Results analysis:

(1) The renal edema incidence in the aristolochic acid-induced zebrafish model group is 100%, and the renal edema incidence in the 1 µM tafamidis treatment group is 80% (FIG. 3), demonstrating that tafamidis exerts a protective effect on the kidney.
(2) The glomerular filtration rate in the normal zebrafish group is 100%, the glomerular filtration rate in the aristolochic acid-induced zebrafish model group is 239%, and the glomerular filtration rate in the 1 µM tafamidis treatment group is 158%, indicating that tafamidis serves a function of improving glomerular filtration (FIG. 4).
(3) The cardiac red blood cell count in the normal zebrafish group is 100%, the cardiac red blood cell count in the aristolochic acid-induced zebrafish model group is 46%, the cardiac red blood cell count in the 0.1 µM tafamidis treatment group is 60%, and the cardiac red blood cell count in the 1 µM tafamidis treatment group is 65%, indicating that tafamidis exerts a significant effect in alleviating renal anemia (FIG. 5).

In an aristolochic acid-induced zebrafish chronic kidney disease model, it is confirmed that tafamidis exerts effects of increasing erythropoiesis and alleviating renal injury,

### Embodiment 4: 5/6 nephrectomy (5/6Nx) rat model

Male Sprague Dawley (SD) rats are used to establish a renal injury-induced anemia model through 5/6 nephrectomy surgery to investigate the erythropoiesis-promoting effect of tafamidis. After a one-week acclimation period, the rats are anesthetized with isoflurane, and the left kidney is exposed through a midline abdominal incision. Ligatures are placed at the upper one-third and lower one-third positions of the kidney, and the corresponding renal tissues outside the ligatures are excised. After a one-week recovery period, the right kidney is excised, with eight rats assigned to the nephrectomy group and the remaining eight rats serving as a non-operated normal control group. At week 4, renal dysfunction in the operated rats is confirmed by assessing serum urea and creatinine levels. Tafamidis and roxadustat are dissolved in a vehicle containing 10% dimethyl sulfoxide (DMSO), 30% polyethylene glycol 400 (PEG 400), 5% Tween-80, 1% hydrochloric acid (113.3 mM), 9% 0.1 N sodium hydroxide, and 45% normal saline. The nephrectomized rats are randomly divided into a 5/6Nx group, a tafamidis treatment group, and a roxadustat treatment group (8 rats per group), ensuring comparable baseline body weights among groups. Starting from week 6, the tafamidis treatment group and the roxadustat group are orally administered the corresponding compound at 10 mg/kg daily (10 mg orally per day per 1 kg body weight of rat) for four weeks, while the sham-operated group and the 5/6 nephrectomy model (5/6Nx) rats are administered the vehicle based on the same dosing regimen. Hematological analysis is performed weekly, and urine samples are collected on the day following the final treatment, so as to determine urinary total protein (UTP) and urinary creatinine (UCREA). Hematological analysis is performed using the Sysmex Europe XN-2000 automated hematology analyzer, and urinalysis is performed using the Cobas C501 automated analyzer, both using commercially available kits in accordance with the manufacturers' instructions. After sample collection, the rats are euthanized. Statistical significance is determined by one-way analysis of variance (ANOVA), and then a Dunnett's test is performed for comparison with the 5/6Nx group (*P < 0.05, **P < 0.01, ***P < 0.001).

### Results analysis:

(1) Hematological analysis shows that, compared with the 5/6Nx group, the 10 mg/kg tafamidis treatment group exhibits a significant increase in red blood cell (RBC) count at week 4 (7.7 ± 0.3 × 10¹² cells/L vs. 8.2 ± 0.3 × 10¹² cells/L, p < 0.05, panel A in FIG. 6). Comparative analysis with the HIF-PHD inhibitor roxadustat shows significant differences in safety profile between the two. Roxadustat at 10 mg/kg shows notable erythrocytosis on day 14, with RBC levels exceeding the physiological range of the healthy control group, suggesting that excessive erythropoiesis may pose a thromboembolic risk.
(2) Renal function evaluation further highlights the therapeutic advantages of tafamidis: urinalysis shows that UTP excretion levels in the tafamidis treatment group remain at baseline levels, whereas those in the roxadustat treatment group increase significantly (an 3.3-fold increase compared with the 5/6Nx group, p < 0.01; panels B and C in FIG. 6), suggesting that roxadustat may have caused renal injury. In contrast, the tafamidis treatment group shows essentially no effect on UCREA, and although the mean UTP value increases, the increase is not statistically significant. These results indicate that tafamidis exerts significant erythropoiesis-promoting activity and demonstrates a better safety profile than roxadustat although without exhibiting significant renal repair activity.

### Embodiment 5: Cisplatin-induced renal injury and anemia rat model

### 5.1 Animal model establishment, drug administration, and sample collection

Male Sprague-Dawley (SD) rats are allowed a one-week acclimation period, after which cisplatin (6 mg/kg) is administered through tail vein injection. A second injection is given one week later. Two weeks after the first administration, successful establishment of a renal disease model is confirmed by measuring serum creatinine and urea levels. The established rat model is randomly divided into four groups: a control group (n = 7), a cisplatin group (n = 6), a roxadustat group (n = 6), and a tafamidis group (n = 6). The roxadustat group and tafamidis group are orally administered the corresponding compound (roxadustat or tafamidis) at 10 mg/kg daily for four consecutive weeks. A corresponding solvent is administered to the control group and cisplatin group rat models based on the same dosing regimen. Urine samples are collected on the day following the final treatment, so as to measure UTP and UCREA. Hematological analysis is performed using a Mindray BC-5150 automated hematology analyzer, and urinalysis is performed using a Mindray BS-2000M automated biochemical analyzer, both using commercially available kits according to the manufacturers' instructions. After sample collection, the rats are euthanized. Statistical significance is determined by one-way analysis of variance (ANOVA), and then a Dunnett's test is performed for comparison with the cisplatin group (*P < 0.05, **P < 0.01, ***P < 0.001).

### 5.2 Renal histopathology and immunohistochemical studies

Left kidney samples collected from cisplatin-induced rats are fixed in 4% paraformaldehyde, dehydrated, embedded in paraffin, and sectioned. The tissue sections are 4 µm thick, and are subsequently subjected to histochemical staining such as hematoxylin-eosin (H&E), periodic acid-Schiff (PAS), and Masson's trichrome staining. After staining, the sections are scanned using advanced three-dimensional histological imaging technology, and high-resolution images are captured using a Pannoramic DESK/MIDI/250/1000 system. To evaluate renal tubular injury, 10 fields are randomly selected from each sample, and examined at a 40× magnification. The degree of renal tubular injury is assessed using a semi-quantitative scoring system (Paller score): 0 (no injury), 1 (<25% injury), 2 (25% to 50% injury), 3 (50% to 75% injury), and 4 (>75% injury). In addition, the entire tissue section is assessed at a 0.7× magnification to determine the extent of collagen fiber deposition shown by Masson's trichrome staining. Semi-quantitative analysis of the collagen fiber area is performed using Image-Pro Plus 6.0 software (Media Cybernetics, Rockville, MD, USA). For glomerular evaluation, 20 glomeruli are randomly selected from each rat and analyzed at a 63× magnification, using mesangial matrix expansion shown by PAS staining as an indicator. Semi-quantitative analysis of the mesangial matrix is likewise performed using Image-Pro Plus 6.0 software. Statistical significance is determined by one-way analysis of variance (ANOVA), and then a Dunnett's test is performed for comparison with the cisplatin group (*P < 0.05, **P < 0.01, ***P < 0.001).

Immunofluorescence staining is performed on 4 µm-thick kidney tissue sections. The sections are deparaffinized and rehydrated through ethanol, and then is subjected to microwave treatment in a 0.01 mol/L sodium citrate buffer (pH 6.0) for antigen retrieval. Subsequently, the sections are incubated overnight at 4 °C with a primary antibody against α-SMA (19245S, Cell Signaling Technology). After incubation, the sections is incubated at room temperature for 30 minutes with an HRP-conjugated secondary antibody (PK10009, Proteintech Group), and localization is performed using a DAB kit. After staining, the sections are scanned using advanced three-dimensional histological imaging technology, and high-resolution images are captured using a Pannoramic DESK/MIDI/250/1000 system. The entire tissue section is assessed at a 0.7× magnification to determine an α-SMA-positive region. Semi-quantitative analysis of the α-SMA expression area is performed using Image-Pro Plus 6.0 software. Statistical significance is determined by one-way analysis of variance (ANOVA), and then a Dunnett's test is performed for comparison with the cisplatin group (*P < 0.05, **P < 0.01, ***P < 0.001).

### 5.3 Western blot analysis

Kidney tissues are lysed in an RIPA buffer containing a protease inhibitor. Protein extracts are separated by electrophoresis in a 10% SDS-PAGE gel and subsequently transferred onto a PVDF membrane. The membrane is blocked in 5% skimmed milk powder for 1 hour and then incubated overnight at 4 °C with primary antibodies. The primary antibodies include α-SMA (19245S, Cell Signaling Technology), EPO (ab226956, Abcam), and NADPH (5174S, Cell Signaling Technology). The membrane is then incubated with the corresponding secondary antibody (ab6721, Abcam). After washing, images are detected using the GE AI600. Signal intensities are quantified and normalized to NADPH as an internal control.

Results analysis: (1) Hematological analysis at week 4 shows that, compared with the cisplatin group, tafamidis significantly improves erythropoiesis-related parameters, as shown in Table 1:

**Table 1**

| | Cisplatin group | Tafamidis group |
|---|---|---|
| Red blood cells (RBCs, unit: cells/L, p < 0.001; panel A in FIG. 7) | 6.3 ± 0.3 ×10¹² | 8.1 ± 0.4 × 10¹² |
| Hemoglobin (HGB, unit: g/L, p < 0.05; panel B in FIG. 7) | 127.7 ± 4.7 | 161.8 ± 9.3 |
| Hematocrit (HCT, p < 0.001; panel C in FIG. 7) | 46.2 ± 1.8% | 58.4 ± 2.9% |

Gross observation of kidney tissues shows that the kidneys in the normal control group exhibit a healthy red color, while those in the cisplatin group show obvious white discoloration and edema. In contrast, the kidneys in the roxadustat group appear purplish red, whereas the kidneys in the tafamidis group exhibit a normal red color, indicating improved renal health (panel D in FIG. 7). In summary, tafamidis shows a significant therapeutic effect in treating renal anemia.

(2) Urinalysis shows that roxadustat increases UTP levels (p < 0.05) (panel A in FIG. 8), indicating potential nephrotoxicity. In contrast, tafamidis increases UCREA levels (p < 0.05) (panel B in FIG. 8) while reducing the UTP/UCREA ratio (p < 0.05) (panel C in FIG. 8), indicating that tafamidis exerts a significant therapeutic effect in treating renal injury.

(3) Periodic acid-Schiff stain (PAS), Masson's trichrome, and hematoxylin-eosin (H&E) staining confirm that cisplatin causes severe renal pathological changes, including glomerular mesangial expansion, interstitial fibrosis, and inflammatory cell infiltration (panels A, C, and E in FIG. 9). Semi-quantitative scoring confirms that tafamidis significantly alleviates cisplatin-induced renal injury (panels B, D, and F in FIG. 9). Compared with roxadustat, tafamidis treatment significantly reduces inflammatory infiltration, renal tubular vacuolar degeneration, and renal fibrosis.

(4) Immunohistochemical results (panels G and H in FIG. 9) show that tafamidis significantly reduces α-smooth muscle actin (α-SMA) protein levels. Western blot analysis shows that tafamidis significantly reduces α-SMA protein expression while increasing erythropoietin (EPO) protein levels (panel I in FIG. 9).

These results confirm that tafamidis exhibits potent therapeutic effects in alleviating cisplatin-induced renal injury and renal anemia.

### Embodiment 6: Unilateral ureteral obstruction (UUO)-induced mouse model of renal injury and anemia

### 6.1 Animal model establishment, drug administration, and sample collection

Animal model establishment: Male Balb/c mice are anesthetized with isoflurane and then secured to an operating table with medical tape. After preparation of an abdominal midline incision site, the surgical part is disinfected with 75% ethanol-iodine tincture-ethanol. Subsequently, an incision is made along the linea alba of the mouse, and the incision is held open with a laparotomy retractor. The left ureter is isolated using a sterile cotton swab and curved forceps and ligated near the bladder dome using 3-0 suture. One drop of normal saline is added into the abdominal cavity, and then the abdomen is sutured. Following recovery on a heating pad, the mouse is returned to the cage, maintained under normal feeding and drinking conditions, and observed, thereby completing model establishment. For the sham-operated group, all procedures are the same as those of the model group except that no ligation is performed. For the treatment groups in the UUO model, male Balb/c mice, after a one-week acclimation period, are orally administered tafamidis (tafamidis treatment group) or roxadustat (roxadustat treatment group) by gavage for three consecutive days. The dose in the first tafamidis treatment group is 5 mg/kg/day, the dose in the second tafamidis treatment group is 10 mg/kg/day, and the dose in the roxadustat treatment group is 10 mg/kg/day. The drug administration is continued for seven days after unilateral ureteral obstruction surgery. A solvent is administered to the mice in the sham-operated group and the UUO control group based on the same dosing regimen. Serum analysis is performed after the final treatment. Serum analysis is performed using a Mindray BS-2000M automated hematology analyzer with commercially available kits based on the manufacturer's instructions. After sample collection, the rats are euthanized. Statistical significance is determined by one-way analysis of variance (ANOVA), and then a Dunnett's test is performed for comparison with the UUO control group (*P < 0.05, **P < 0.01, ***P < 0.001).

### 6.2 Renal histopathology studies

Isolated left kidney samples from mice are fixed in 4% paraformaldehyde, dehydrated, embedded in paraffin, and sectioned. The tissue sections are 4 µm thick, and are subsequently subjected to histochemical staining such as H&E staining and Masson's trichrome staining. After staining, the sections are scanned using advanced three-dimensional histological imaging technology, and high-resolution images are captured using a Pannoramic DESK/MIDI/250/1000 system. H&E staining is analyzed to evaluate renal tubular injury. 10 fields are randomly selected from each sample, and examined at a 40× magnification. The degree of renal tubular injury is assessed using a semi-quantitative scoring system (Paller score): 0 (no injury), 1 (<25% injury), 2 (25% to 50% injury), 3 (50% to 75% injury), and 4 (>75% injury). In addition, the entire tissue section is assessed at a 0.7× magnification to determine the extent of collagen fiber deposition shown by Masson's trichrome staining. Semi-quantitative analysis of the collagen fiber area is performed using Image-Pro Plus 6.0 software. Statistical significance is determined by one-way analysis of variance (ANOVA), and then a Dunnett's test is performed for comparison with the UUO control group (*P < 0.05, **P < 0.01, ***P < 0.001).

The relevant results are recorded in Table 2 below.

Results Analysis: Serum biochemical analysis shows that serum creatinine is significantly elevated in the UUO group compared with normal rats. In contrast, creatinine levels in the treatment group show no significant change compared with the UUO group. Overall analysis of urea levels shows that, compared with the urea level in the UUO control group (6.26 ± 0.14 mmol/L), both the 10 mg/kg roxadustat treatment group (5.36 ± 0.10 mmol/L, P < 0.01) and the second tafamidis 10 mg/kg treatment group (5.28 ± 0.29 mmol/L, P < 0.05) exhibit significantly lower urea levels, indicating that both roxadustat and tafamidis exert renal protective effects (panels A and B in FIG. 10).

Gross observation shows that the first tafamidis group (5 mg/kg tafamidis) significantly alleviates the pallor of the obstructed kidney (panel C in FIG. 10), suggesting potential efficacy in alleviating renal ischemia associated with ureteral obstruction.

Histopathological evaluation using H&E staining and the Paller scoring system (panels D and E in FIG. 10) shows that, compared with the UUO control group (4.56 ± 0.19), in both the first tafamidis group (5 mg/kg; 2.85 ± 0.83, P < 0.05) and the second tafamidis group (10 mg/kg; 2.93 ± 0.35, P < 0.05), different dosages of tafamidis significantly alleviate renal tubular injury, with markedly lower pathological scores. In contrast, the 10 mg/kg roxadustat treatment group (4.78 ± 0.09, P = 0.98) still exhibits severe inflammatory infiltration and tubular injury.

Masson's trichrome staining analysis (panels F and G in FIG. 10) shows that, compared with the UUO control group (26.0 ± 1.9%), interstitial fibrosis is significantly reduced in both the first tafamidis group (5 mg/kg; 17.2 ± 2.3%, P < 0.05) and the second tafamidis group (10 mg/kg; 17.2 ± 2.6%, P < 0.05). In contrast, the 10 mg/kg roxadustat treatment group (21.8 ± 1.6%, P = 0.37) shows no significant reduction in fibrosis.

These results confirm that tafamidis exerts significant therapeutic effects on renal injury and ischemia induced by unilateral ureteral obstruction.

**Table 2**

| | UUO control group | Roxadustat treatment group | Tafamidis treatment group | |
|---|---|---|---|---|
| | | | 5 mg/kg tafamidis | 10 mg/kg tafamidis |
| Urea level (mmol/L) | 6.26 ± 0.14 | 5.36 ± 0.10, P < 0.001 | 5.67 ± 0.23, P = 0.10 | 5.28 ± 0.29, P < 0.05 |
| Pathological score for renal tubular injury | 4.56 ± 0.19 | 4.78 ± 0.09, P = 0.98 | 2.85 ± 0.83, P < 0.05 | 2.93 ± 0.35, P < 0.05 |
| Reduction in fibrosis | 26.0 ± 1.9% | 21.8 ± 1.6%, P = 0.37 | 17.2 ± 2.3%, P < 0.05 | 17.2 ± 2.6%, P < 0.05 |

The methods disclosed in this application have been described with reference to preferred embodiments. Evidently, those skilled in the art can implement and apply the technical solutions of this application by modifying or appropriately varying and combining the methods and applications described herein without departing from the subject-matter, essence, and scope of this application. Those skilled in the art may implement the technical solutions by appropriately modifying some process parameters with reference to the teachings hereof. It is particularly important to note that all similar replacements and modifications are apparent to those skilled in the art and are deemed to fall within the scope of protection of this application.

## Claims

1. A use of tafamidis or a pharmaceutically acceptable salt thereof, or a pharmaceutical composition containing tafamidis or a pharmaceutically acceptable salt thereof in preparing a drug for preventing, alleviating, or treating diseases associated with HIF-2α activity.

2. The use according to claim 1, **characterized in that** the pharmaceutically acceptable salt of tafamidis is an acid addition salt or a base addition salt.

3. The use according to claim 1, **characterized in that** the pharmaceutically acceptable salt of tafamidis is at least one selected from the following structures:

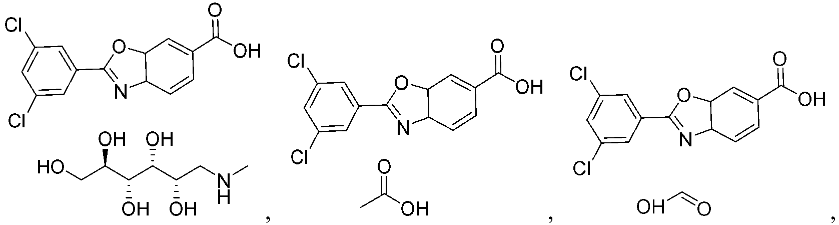

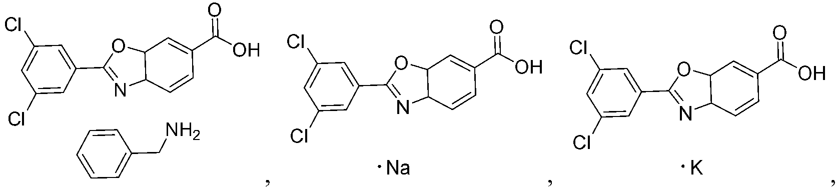

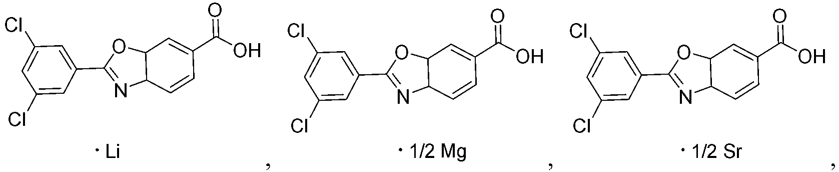

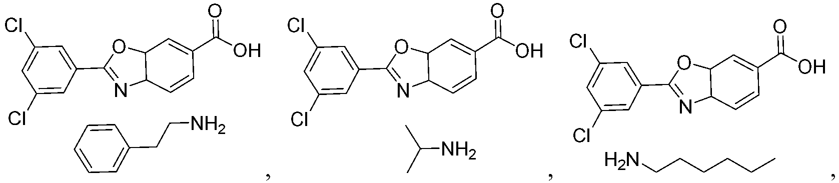

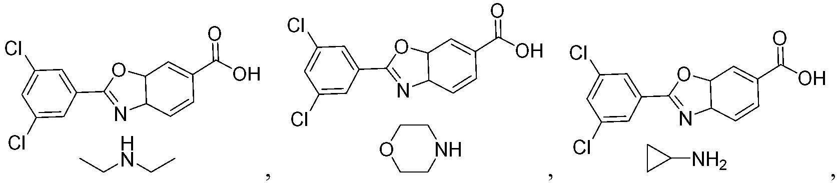

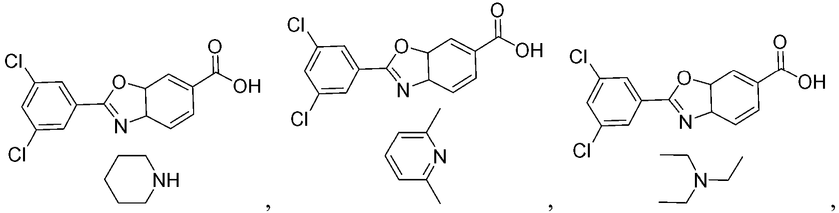

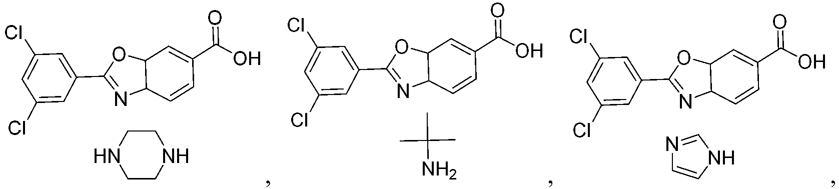

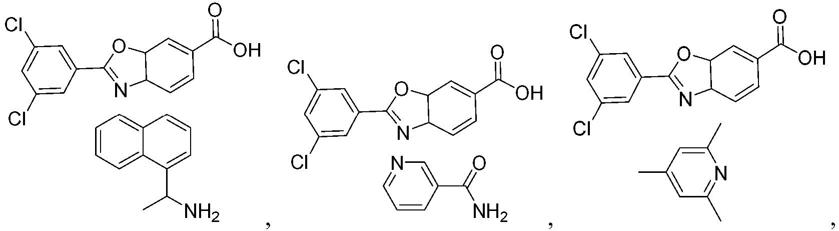

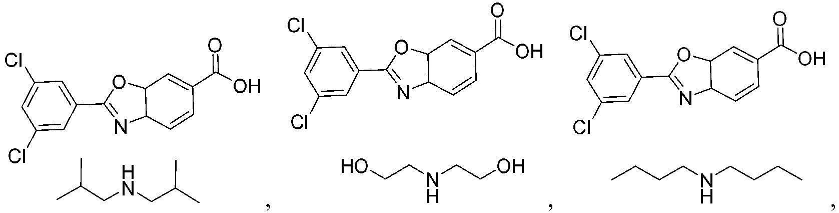

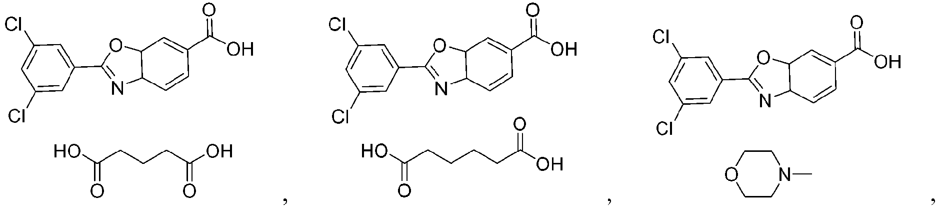

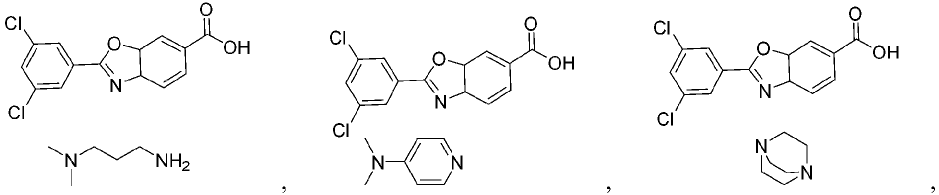

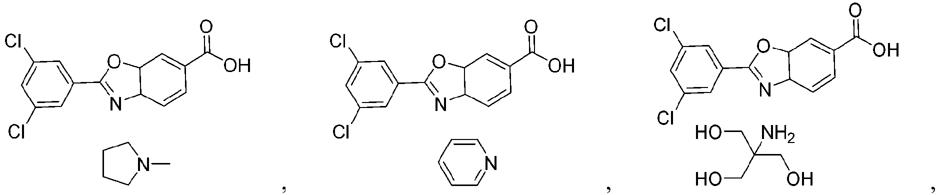

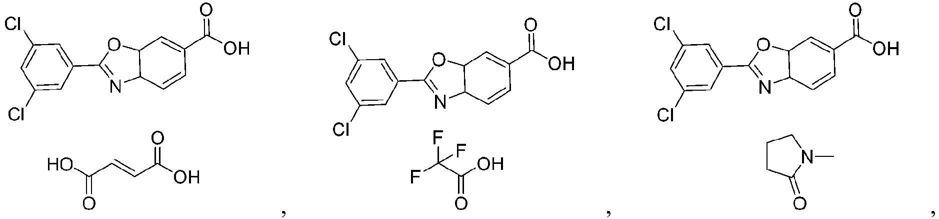

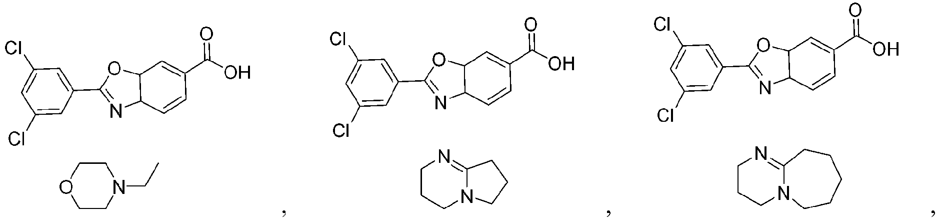

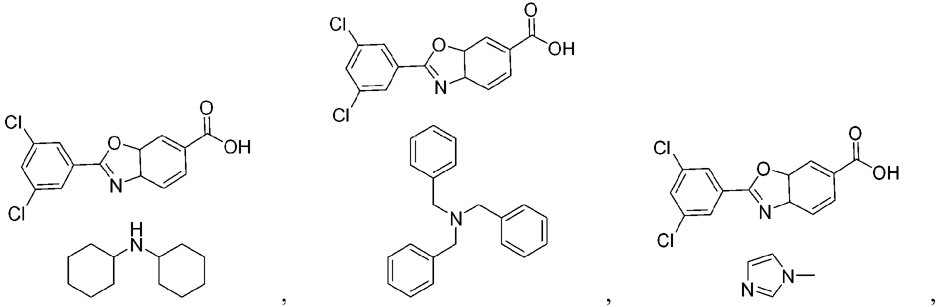

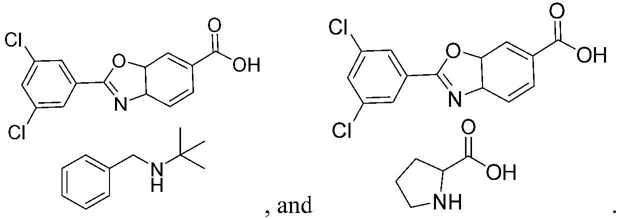

4. The use according to claim 1, **characterized in that** the pharmaceutically acceptable salt of tafamidis is

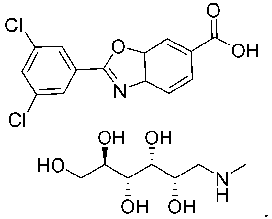

5. The use according to any one of claims 1 to 4, **characterized in that** the pharmaceutical composition further comprises a pharmaceutically acceptable excipient.

6. The use according to any one of claims 1 to 5, **characterized in that** the disease associated with HIF-2α activity comprises hematopoietic disorders, anemia, postoperative surgery-related ischemia and sequelae thereof, postoperative wound healing, chronic kidney diseases, cardiovascular diseases, infections, inflammatory diseases, cancers, impairment of health status occurring during cancer treatment, or sequelae of acute and prolonged cerebral ischemia.

7. The use according to any one of claims 1 to 5, **characterized in that** the disease associated with HIF-2α activity comprises: renal anemia; primary anemia; anemia associated with a neoplastic disease; chemotherapy-induced anemia; blood-loss-induced anemia; iron-deficiency-induced anemia; vitamin-deficiency-induced anemia; hypoplastic and aplastic anemia; hemolytic anemia; anemia caused by impaired iron utilization (anemia caused by iron utilization failure) or due to other endocrine disorders (such as hypothyroidism); ischemia and sequelae thereof caused by cardiac interventions using a heart-lung machine (such as bypass surgery and heart valve transplantation), carotid interventions, aortic interventions, and interventions involving opening or penetrating a skull using an instrument; primary glomerulonephritis; hypertensive renal arteriolosclerosis; diabetic nephropathy; secondary glomerulonephritis; tubulointerstitial diseases (chronic pyelonephritis, chronic uric acid nephropathy, obstructive nephropathy, drug-induced nephropathy, and the like); ischemic nephropathy; hereditary nephropathy (polycystic kidney, hereditary nephritis); cardiac insufficiency; coronary heart disease; angina pectoris; myocardial infarction; stroke; arteriosclerosis; primary, pulmonary and malignant hypertension and peripheral arterial occlusive disease; HIV infection; rheumatoid arthritis; diseases of a rheumatic spectrum or other disease forms considered as autoimmune diseases following the use of cytostatics, antibiotics, or radiotherapy; or impairment of health status occurring during pharmacological treatment of such diseases (such as stroke and birth asphyxia);
or, the disease associated with HIF-2α activity comprises anemia, ischemia or an ischemic disease, vascular diseases, angina pectoris, myocardial infarction, metabolic disorders, or cancers;
or, the disease associated with HIF-2α activity comprises renal anemia and/or nephropathy.

8. The use according to any one of claims 1 to 7, **characterized in that** the tafamidis or a pharmaceutically acceptable salt thereof, or a pharmaceutical composition containing tafamidis or a pharmaceutically acceptable salt thereof, achieves prevention, alleviation, or treatment of diseases associated with HIF-2α activity by upregulating downstream VEGF genes regulated by HIF-2α.

9. The use according to any one of claims 1 to 7, **characterized in that** the tafamidis or a pharmaceutically acceptable salt thereof, or a pharmaceutical composition containing tafamidis or a pharmaceutically acceptable salt thereof, achieves prevention, alleviation, or treatment of diseases associated with HIF-2α activity by upregulating downstream EPO genes regulated by HIF-2α.
